# EUROPEAN PATENT SPECIFICATION

(11) **EP 4 211 432 B1**
(45) Date of publication and mention of the grant of the patent: **29.07.2026**
(21) Application number: 21786667.2
(22) Date of filing: 07.09.2021
(51) Int. Cl.: G01S 15/89, A61B 5/00, G01H 9/00, G01S 7/52

(54) **SYNTHETIC APERTURE IMAGING SYSTEMS AND METHODS USING MIXED ARRAYS**
SYNTHETISCHE APERTURABBILDUNGSSYSTEME UND VERFAHREN MIT GEMISCHTEN ARRAYS
SYSTÈMES ET PROCÉDÉS D'IMAGERIE À OUVERTURE SYNTHÉTIQUE UTILISANT DES RÉSEAUX MIXTES

(30) Priority: 08.09.2020 US 202063075727 P
(43) Date of publication of application: 19.07.2023
(73) Proprietor: Deepsight Technology, Inc., Los Altos, CA 94024 (US)
(72) Inventor: ZHAO, Danhua, Los Altos CA94024 (US); ZHU, Liren, Los Altos CA 94024 (US)
(74) Representative: Slingsby Partners LLP
(86) International application number: PCT/US2021/049226
(87) International publication number: WO 2022/055843

(56) References cited:
- EP-A1- 3 126 872
- US-A1- 2008 095 490

## Description

### TECHNICAL FIELD

The present disclosure generally relates to the field of ultrasound imaging, and in particular to methods and devices that enable forming a synthetic aperture by combining signals from a mixed array including an array of optical resonators and other sensors. The methods and devices disclosed herein include optical resonators that have high sensitivity and high operational bandwidth for improved imaging performance.

### BACKGROUND OF THE INVENTION

Ultrasound sensing is used in various industries including medical imaging and medical diagnosis due to a number of advantages. For example, ultrasound sensing utilizes ultrasound signal which has a remarkable penetration depth. Moreover, ultrasound imaging is known to be an advantageously non-invasive form of imaging, as it is based on non-ionizing radiation.

Various known ultrasound transducers used in ultrasound imaging have numerous drawbacks. For example, some ultrasound transducers are made of piezoelectric material, such as lead zirconate titanate (PZT). However, the 6 dB bandwidth of PZT materials is generally limited to only about 70%. Certain composite PZT materials have a slightly increased bandwidth, but still only achieve a bandwidth of up to about 80%. As another example, single crystal materials have increasingly been used in an effort to improve performance of ultrasound probes, but have lower Curie temperatures and are brittle. Another type of transducer material is silicon, which can be processed to build Capacitive Micromachined Ultrasound Transducer (CMUT) probes that can have increased bandwidth. However, CMUT probes are not very sensitive or reliable. Moreover, CMUT probes have several operational limitations. For example, CMUT probes are nonlinear sensors and, therefore, are not generally suitable for harmonic imaging. In addition, CMUT probes require an additional bias voltage to operate properly. Thus, there is a need for ultrasounds probes that include sensor with higher bandwidth and sensitivity.

US2008095490A1 describes a polymer waveguide resonator device for high-frequency ultrasound detection having an optical resonator coupled to a straight optical waveguide which serves as input and output ports. EP3126872A1 describes an apparatus and method of imaging an imaging region that employ an acoustic transducer array to produce image data for the imaging region, wherein there are one or more obstructions between the acoustic transducer array and at least a portion of the imaging region.

### BRIEF SUMMARY OF THE INVENTION

The present invention is as set out in the appended claims. Generally, in some variations, an apparatus for imaging a target, include: a transmitter for transmitting acoustic waves; one or more array elements of a first type forming a first sub-aperture, and one or more array elements of a second type different from the first type and forming a second sub-aperture; the second type being an optoacoustic transducer. The array elements of the first and second types configured to detect acoustic echoes corresponding to the transmitted acoustic waves The first sub-aperture receives a first signal having a first phase and the second sub-aperture receives a second signal having a second phase. The apparatus further includes a front-end, comprising a receive beamformer, and configured to obtain a combined signal for forming an image; the combined signal is from a synthesized aperture comprising the first sub-aperture and the second sub-aperture. In some variations, the front-end may be configured to generate a synthesized aperture using one or more aspects of the methods as described herein.

Generally, in some variations, a method of acousto-optic imaging may include receiving a first signal from a first sub-aperture of a sensor array, wherein the first sub-aperture includes one or more array elements of a first type. The method may further include receiving a second signal from a second sub-aperture of the sensor array, wherein the second sub-aperture includes one or more array elements of a second type different from the first type. The second type is an optoacoustic transducer. The array elements of the first and second types configured to detect acoustic echoes corresponding to the transmitted acoustic waves. The method further includes combining, using a receive beamformer, the first signal and the second signal to obtain a combined signal for forming an image; the combined signal is from a synthesized aperture comprising the first sub-aperture and the second sub-aperture of the sensor array.

In some variations of the apparatus and method, the first type of array element may be a non-optical sensor such as an acoustic transducer (e.g., piezoelectric transducer or capacitive micromachined ultrasonic transducer (CMUT) sensor) configured to transmit acoustic waves and the second type of array element may be an optical sensor such as a whispering gallery mode (WGM) sensor. The optical sensor can be/include a microsphere resonator, a microtoroid resonator, a microring resonator (e.g., having a circular cross-sectional shape or non-circular cross-sectional shape such as a racetrack or elliptical), a microbubble resonator, a photonic integrated circuit (PIC) resonator, and/or a micro-disk resonator. In some instances, the array elements of the first and second types may be configured to detect acoustic echoes corresponding to the transmitted acoustic waves.

In some variations, the method may further include phase matching the first signal and the second signal. To phase match the signals, a first delay may be applied to the first signal and/or a second delay may be applied to the second signal. In some instances, the first delay and/or the second delay may be determined based at least in part on a difference between a first propagation time from the one or more array elements of a first type to a medium being imaged and a second propagation time from the one or more array elements of a second type to the medium. Additionally, or alternatively, the first delay and/or the second delay may be determined based on a thickness and acoustic velocity of an acoustic lens and/or a thickness and acoustic velocity of an acoustic matching layer. The first delay and/or the second delay may be presented as a delay profile that takes into account various differences between each array element and/or sub-elements .

In some variations, the method may further include filtering the first signal and/or the second signal to reduce noise in the signals and/or to match frequency range of the signals. The filter may include a band-pass filter, a low-pass filter, a high-pass filter, a digital filter, and/or the like. In some variations, the method may further include amplifying the first signal and/or the second signal by an amplification gain to amplitude match the first signal and the second signal. The amplification gain may be a preset value and/or determined based on imaging depth. The amplification gain can include a constant value or include tensor of amplification gain values that provide a specific gain for each array element.

The ultrasound sensor array may be a 1 dimensional (1D) array, a 1.25 dimensional (1.25D) array, a 1.5 dimensional (1.5D) array, a 1.75 dimensional (1.75D) array, or a 2 dimensional (2D) array. In some variations, the one or more array elements of the first type and the one or more array elements of the second type may be arranged in the 1.25D array or the 1.5D array. Each of the 1.25D array or the 1.5D array can include a first row and a second row. The first row can include a first number of array elements and the second row can include a second number of array elements. In some instances, the first number of array elements in the first row can be equal to the second number of array elements in the second row. For example, the first row and the second row, each may include 128 array elements. In some instances, the first number of array elements in the first row can be different from the second number of array elements in the second row. For example, the first row may include 128 array elements, while the second row may include 192 array elements.

In some variations, the first signal may include a combination of signals originating from multiple array elements of the first type. Additionally or alternatively, the second signal may include a combination of signals originating from multiple array elements of the second type. Combining signals from array elements of similar type with a close distance from one another can reduce dimensionality of the mixed array (e.g., from a 1.5D array to a 1D array). As a result, the mixed array may require fewer filters and/or amplifiers.

In some variations, the method may include frequency matching, amplitude matching, and phase matching the first signal and the second signal in any suitable order. For example, the method may include frequency matching, followed by amplitude matching and then phase matching the first signal and the second signal. As another example, the method may include phase matching, amplitude matching, and frequency matching the first signal and the second signal in that order. After performing frequency matching, amplitude matching, and phase matching for each array element type separately, the first signal and the second signal can be combined. The combination can involve a coherent combination.

In some variations, the method may include selecting the first sub-aperture for transmitting acoustic signals and a combination of the first sub-aperture and the second sub-aperture for receiving acoustic echoes in response to the acoustic signals. In some variations, the method may include selecting an element from the one or more array elements of the first type for transmitting acoustic signals and a combination of the first sub-aperture and the second sub-aperture for receiving acoustic echoes in response to the acoustic signals. In some variations, the method may include selecting an angle (e.g., steering angle) for transmitting acoustic signals and/or receiving acoustic echoes. The selection processes above may be iteratively repeated until all sub-apertures, array elements, and/or angles have been fully covered.

In some variations, the one or more array elements of the second type can include an optical sensor(s) embedded in a polymer structure. The optical sensor(s) may be optically coupled to an optical fiber to transmit a set of optical signals to a photodetector. The optical sensor(s) may be configured to alter the optical signals in response to the acoustic echoes.

### BRIEF DESCRIPTION OF THE DRAWINGS

FIG. 1 is a block diagram of an exemplary synthetic aperture imaging system with a mixed array.
FIG. 2 is a block diagram of an exemplary front-end of a synthetic aperture imaging system with a mixed array.
FIG. 3 is a block diagram of an exemplary front-end of a synthetic aperture imaging system with a mixed array.
FIG. 4 is a block diagram of an exemplary probe of a synthetic aperture imaging system with a mixed array.
FIG. 5 is a schematic illustration of an exemplary mixed array of a synthetic aperture imaging system.
FIG. 6 is a schematic illustration of an exemplary mixed array of a synthetic aperture imaging system.
FIG. 7 is a schematic illustration of an exemplary mixed array of a synthetic aperture imaging system.
FIG. 8 is a schematic illustration of an exemplary mixed array of a synthetic aperture imaging system.
FIG. 9 is a schematic illustration of an exemplary mixed array of a synthetic aperture imaging system.
FIG. 10 is a schematic illustration of an exemplary mixed array of a synthetic aperture imaging system.
FIG. 11 is a flowchart of an exemplary method of performing synthetic aperture imaging using a mixed array.
FIG. 12 is a flowchart of an exemplary method of performing synthetic aperture imaging using a mixed array.
FIG. 13 is a flowchart of an exemplary method of performing synthetic aperture imaging using a mixed array.
FIG. 14 is a block diagram of an exemplary method of performing synthetic aperture imaging using a mixed array.
FIG. 15 is a block diagram of an exemplary method of performing synthetic aperture imaging using a mixed array.
FIG. 16 is a block diagram of an exemplary method of performing synthetic aperture imaging using a mixed array.
FIG. 17 is a block diagram of an exemplary method of performing synthetic aperture imaging using a mixed array.
FIG. 18 shows exemplary signals generated by two types of sensors in a mixed array.
FIG. 19 shows exemplary frequency responses of two bandpass filters tailored for two types of sensors in a mixed array.
FIG. 20 shows exemplary mixed array windows and their corresponding beamplots.
FIG. 21 shows exemplary synthesized aperture windows for mixed arrays and their corresponding beamplots.
FIG. 22 shows an exemplary delay profile for a uniform array and an exemplary delay profile for a mixed array.

### DETAILED DESCRIPTION

Non-limiting examples of various aspects and variations of the invention are described herein and illustrated in the accompanying drawings.

Described herein are methods and devices for synthetic aperture imaging using ultrasound probes with mixed arrays that includes array elements of multiple different types. Mixed arrays described herein include one or more array elements of a first type and one or more array elements of a second type (e.g., optical sensors such as WGM optical resonators and/or the like) different from the first type. The optical sensors have high sensitivity and broad bandwidth in reception of ultrasound signals compared to other types of ultrasound sensors. The one or more array elements of the first type (e.g., transducers, or a non-optical sub-array) may be used to form a first set of signals. In parallel, the one or more array elements of the second type (e.g., optical sensors in an optical sub-array) is used to detect acoustic echoes that can be used to form a second set of signals. The second set of signals that are generated by highly sensitive and broadband optical sensors may be used independently or can be combined with the first set of signals to form an even further improved image. Because of the high sensitivity and broad bandwidth of optical sensors, the image produced by optical sensors may have improved spatial resolution, improved contrast resolution, improved penetration depth, improved signal-to-noise ratio (NSR), improved tissue harmonic imaging, and/or improved Doppler sensitivity.

The optical sensors do not generate ultrasound signals and therefore are used together in mixed arrays with other transducers (e.g., piezoelectric transducer, CMUT, and/or the like) that do generate ultrasound signals. The mixed arrays can be arranged in various configurations and include sensor elements with various noise levels, amplitude responses, phase delays, frequency ranges, and/or the like. Consequently, beamforming methods and devices that are generally used for probes with one type of sensor cannot be used for probes that use mixed arrays of multiple types of sensors.

For each mixed array configuration, beamforming methods and algorithms may be tailored to fit the mixed array configuration. Since both the non-optical sub-array and the optical sub-array may be used for receiving ultrasound echo signals, the receive aperture of the mixed array can be divided into multiple sub-apertures. For example, a first receive sub-aperture (also referred to as a "non-optical aperture") may include one or more sensors that are not optical sensors. Additionally, a second receive sub-aperture (also referred to as "optical sensor aperture") may include one or more optical sensors. The receive aperture may include additional sub-apertures (e.g., third sub-aperture, fourth sub-aperture, etc.). Signals received from the sub-apertures may be combined together by a receive beamformer of an imaging system to produce a synthesized aperture, as further described below.

Using the beamformer for synthetic aperture ultrasound imaging has a number of advantages. For example, the synthetic aperture ultrasound imaging can increase an aperture size without increasing a number of system channel count. Additionally, the synthetic aperture ultrasound imaging can increase frame rate of ultrasound imaging without reducing a line density in an image produced by synthetic aperture ultrasound imaging. As another example, the synthetic aperture ultrasound imaging can improve image quality by realizing dynamic focusing for both transmitting and receiving.

### Synthetic Aperture Imaging Systems

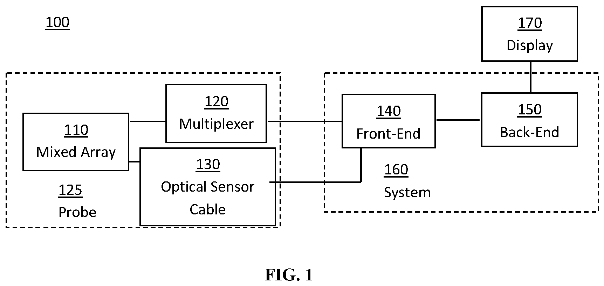

FIG. 1 is a block diagram of an exemplary synthetic aperture imaging system 100 with a mixed array. The synthetic aperture imaging system 100 includes a probe 125, an imaging system 160, and a display 170. The probe 125 can be coupled (e.g., communicatively coupled) to the imaging system 160. The probe 125 can receive and/or transmit a set of signals (e.g., electrical signals, optical signals, etc.) from/to the imaging system 160. The probe 125 can include a mixed array 110 that can receive and/or transmit a set of signals (e.g., acoustic signals, etc.) from/to a medium to form an ultrasound image. The imaging system 160 can include a front-end 140 and a back-end 150 that may collectively determine physical parameters (e.g., timing, location, angle, intensity, and/or the like) of signals transmitted to the probe (e.g., via one or more transmit channels), and process signals received by the probe 125 (e.g., via one or more receive channels) to form an image. The imaging system 160 can also be communicatively coupled to the display 170 to transmit a set of signals (e.g., electrical signals, electromagnetic signals, etc.) to the display 170. For example, in some variations, the display 170 can be configured to display the image produced by the imaging system 160 (e.g., in a graphical user interface (GUI). Additionally or alternatively, the imaging system 160 may receive signals from the display 170. For example, the display 170 may further include an interactive interface (e.g., touch screen, keyboard, motion sensor, and/or the like) to receive commands from a user of the synthetic aperture imaging system 100, such as to control operation of the synthetic aperture imaging system 100.

As shown in FIG. 1, the probe 125 may include a mixed array 110, a multiplexer 120, and an optical sensor cable 130. The mixed array 110 may include one or more array elements of a first type (non-optical sensors such as PZT transducer, CMUT transducer, etc.) and one or more array elements of a second type (optical sensors such as WGM resonators). The non-optical transducers may be configured to transmit acoustic waves, and in some variations may be configured to additionally receive and detect echo signals in response to transmitted acoustic waves. The optical sensors may be configured to receive and detect echo signals with high sensitivity and broadband response. In some variations, the probe 125 can be configured to iteratively scan across a field of view by using the mixed array 110. Doing so will generate images using the optical sensors and/or the non-optical transducers, as described in further detail below. The non-optical transducers in the mixed array 110 can be operatively coupled to the multiplexer 120 that handles transmitted and/or received electrical signals between the imaging system 160 and the non-optical transducers. The one or more array elements of the second type in the mixed array 110 can be operatively coupled to the optical sensor cable 130 that handles transmitted and/or received optical signals between the imaging system 160 and the optical sensors.

The multiplexer 120 functions to selectively connect individual system channels to desired array elements. The multiplexer 120 may include analog switches. The analog switches may include a large number of high voltage analog switches. Each analog switch can be connected to an individual system channel. As a result, the multiplexer 120 may selectively connect an individual system channel from a set of system channels of the imaging system 160 to a desired transducer element of the mixed array 110.

The optical sensor cable 130 may include a dedicated optical path for transmitting and/or receiving optical signals to and/or from the optical sensors. The optical sensor cable 130 may include one or more optical waveguides such as a fiber optical cable(s) or a coaxial cable(s). Characteristics of the optical sensor cable 130 may depend upon type of the optical signals, type of optical sensors, and/or an arrangement of optical sensors. In some configurations, multiple optical sensors (e.g., the entire sub-array of the optical sensors, or any two or more optical sensors forming a portion thereof) can be optically coupled to a single optical waveguide. Accordingly, signals from multiple optical sensors can be coupled into and communicated by a single optical waveguide. In some configurations, the sub-array of the optical sensors can be optically coupled to an array of optical waveguides in a 1:1 ratio (e.g., each optical sensor may be coupled to a respective optical waveguide). Accordingly, optical signals from the sub-array of the optical sensors can be coupled to and communicated by one or more optical waveguides in the optical sensors cable 130 to the imaging system 160. Furthermore, in some variations the synthetic aperture imaging system 100 may include multiple optical sensor cables constructed as described above.

The imaging system 160 may include a front-end 140 and a back-end 150. Generally, the front-end 140 interfaces with the probe 125 to generate acoustic beams and receive electrical and/or optical signals. The back-end system 153 may include one or more processors to process signals received from the mixed array 110 via the front-end to generate images, a memory operatively coupled to the processor to store the images, and/or a communication interface to present the images to a user (e.g., via graphical user interface).

For example, the display 170 may be operatively coupled to the back-end system 150 of the imaging system 160 to display a set of images generated by the imaging system 160. In some variations, the display 170 may additionally or alternatively include an interactive user interface (e.g., a touch screen) and be configured to transmit a set of commands (e.g., pause, resume, and/or the like) to the imaging system 160. In some variations, the synthetic aperture imaging system 100 may further include a set of one or more ancillary devices (not shown) used to input information to the synthetic aperture imaging system 100 or output information from the synthetic aperture imaging system 100. The set of ancillary device may include, for example, a keyboard(s), a mouse(s), a monitor(s), a webcam(s), a microphone(s), a touch screen(s), a printer(s), a scanner(s), a virtual reality (VR) head-mounted display, a joystick(s), a biometric reader(s), and/or the like (not shown).

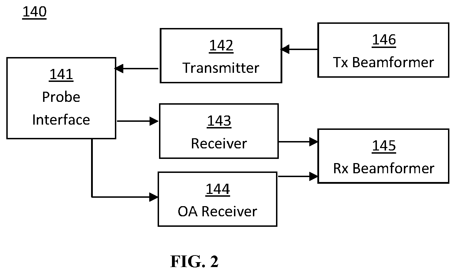

FIG. 2 is a block diagram of an exemplary front-end 140 of a synthetic aperture imaging system 100 with a mixed array 110, where both non-optical sensors and optical sensors may be used to detect ultrasound signals. As shown in FIG. 2, in some variations, the front-end 140 may include a probe interface(s) 141, a transmitter(s) 142, a receiver(s) 143, an optoacoustic receiver(s) 144, a transmit beamformer(s) 146, and a receive beamformer(s) 145. The transmit beamformer 146 may include one or more transmit channels and the receive beamformer 145 may include one or more receive channels. Each transmit or receive channel may be connected (e.g., via a set of electrical wires, via a set of optical waveguides, and/or the like) to an array element of the mixed array 110. For example, the transmit beamformer 146 may include 128 transmit channels and the receive beamformer 145 may include 256 receive channels.

The transmit beamformer 146 may generate various transmit waveforms based on an imaging mode. The waveforms can be amplified by the transmitter 142 before being applied, via the probe interface 141, to elements of the probe 125. The probe interface 141 is responsible for connecting the imaging system 160 to the probe 125 with the mixed array 110, such that the probe 125 may send acoustic signals toward an imaging target. The receiver 143 may receive echo signals detected by the non-optical sensors in response to the acoustic signals as input, process these echo signals to produce a first set of digitalized signals as output, and send such output to the receive beamformer 145. Additionally, the optoacoustic receiver 144 may receive echo signals detected by the optical sensors in response to the acoustic signals as input, process those echo signals to produce a second set of digitalized signals as output, and send such output to the receiver beamformer 145. The receive beamformer 145 uses the first set of signals and the second set of signals to produce receive beams.

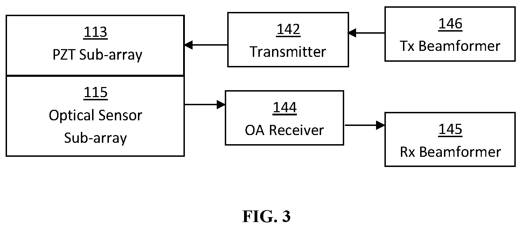

FIG. 3 is a block diagram of an exemplary front-end 140 of a synthetic aperture imaging system 100 with a mixed array 110, where only optical sensors of the mixed array 110 are used to detect ultrasound echo signals (that is, non-optical sensors such as sensors in the PZT sub-array 113 are only used to transmit ultrasound signals and not to detect echo signals). As shown in FIG. 3, the front-end 140 may include a transmitter 142, an optoacoustic receiver 144, a transmit beamformer 146, and a receive beamformer 145. The transmitter 142 can be connected to or operatively coupled (e.g., via the probe interface 141) to non-optical sensors such as a PZT sub-array 113, so as to enable the non-optical array elements to transmit acoustic waves. Optical sensor sub-array 115 of the mixed array may be used to detect echo signals and communicate them to the optoacoustic receiver 144. In contrast in the variation shown in FIG. 2, since no signals are detected by non-optical sensors in the variation shown in FIG. 3, no separate receiver 143 associated with non-optical sensors is required to generate signals based on the acoustic echoes. Accordingly, the receive beamformer 145 uses the signals generated by the optoacoustic receiver 144 to produce receive beams.

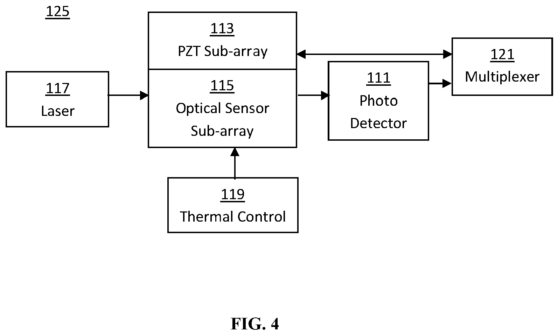

FIG. 4 is a block diagram of an exemplary probe 125 of a synthetic aperture imaging system 100 with a mixed array 110. The probe 125 may include the mixed array 110, a light source(s) 117, a thermal control unit(s) 119, a photodetector(s) 111, and a multiplexer(s) 121. The light source 117 may generate a continuous wave (CW) or pulsed light emission (stimulated emission, spontaneous emission, and/or the like). The light source 117 may further out-couple the light emission to one end of a waveguide medium (e.g., optical fiber, free space, photonic integrated circuit waveguide, and/or the like) that is optically coupled to an optical resonator of an optical sensor sub-array 115. The photodetector 111 receives an out-coupled light from the optical resonator at the other end of the waveguide medium. The out-coupled light generally undergoes phase, amplitude, and/or spectral changes due to presence of the optical resonator and acoustic vibrations (e.g., corresponding to reflected acoustic waves). The thermal control 119 of the probe 125 may maintain a constant temperature for the optical resonators. In some instances the thermal control 119 may be used to stabilize optical response of the optical resonators.

### Mixed Arrays

The mixed array 110 includes an array of sensor elements and may be configured for operation in a 1 dimensional (1D) configuration, a 1.25 dimensional (1.25D) array configuration, a 1.5 dimensional (1.5D) array configuration, a 1.75 dimensional (1.75D) array configuration, or a 2 dimensional (2D) array configuration, such as those as further described below. Generally, dimensionality of the ultrasound sensor array relates to the range of elevation beam width (or elevation beam slice thickness) that is achievable when imaging with the ultrasound sensor array, and how much control the system over the sensor array's elevation beam aperture size, foci, and/or steering throughout an imaging field (e.g., throughout imaging depth). A 1D array has only one row of elements in elevation dimension and a fixed elevation aperture size. A 1.25D array has multiple rows of elements in elevation dimension and a variable elevation aperture size, but a fixed elevation focal point via an acoustic lens. A 1.5D array has multiple rows of elements in elevation dimension, a variable elevation aperture size, and a variable elevation focus via electronic delay control. A 1.75D array is a 1.5D array with additional elevation beam steering capability. A 2D array has large numbers of elements in both lateral and elevation dimensions to satisfy the minimum pitch requirement for large beam steering angles.

In some variations, the synthetic aperture ultrasound imaging system can turn a 1.5D array configuration or a 2D array configuration into a 1D array configuration. The mixed array 110 may include a large number (e.g., 16, 32, 64, 128, 256, 1024, 4096, 8192, 16384, and/or the like) of elements. In some variations, the mixed array 110 may be arranged in a rectangular configuration and may include *N* × *M* elements, where *N* is the number of rows and *M* is the number of columns. The mixed array 110 includes one or more array elements of a first type and one or more array elements of a second type, where the first type may be a transducer or other non-optical sensor configured to transmit ultrasound waves and the second type may be an optical sensor such as a WGM optical resonator. The one or more array elements of the first type and the one or more array elements of the second type may be collectively positioned in a rectangular arrangement, a curved arrangement, a circular arrangement, or a sparse array arrangement. For example, in some variations the mixed array may be similar to any of the mixed arrays described in U.S. Patent App. No. 63/029 044. Furthermore, the mixed array may be configured to perform harmonic imaging as described in U.S. Patent App. No. 63/046 888.

The transducer(s) in the mixed array 110 may include, for example, a lead zirconate titanate (PZT) transducer(s), a polymer thick film (PTF) transducer(s), a polyvinylidene fluoride (PVDF) transducer(s), a capacitive micromachined ultrasound transducer (CMUT) transducer(s), a piezoelectric micromachined ultrasound transducer (PMUT) transducer(s), a photoacoustic sensor(s), a transducer (s) based on single crystal materials (e.g., LiNbO₃(LN), Pb(Mg_{1/3}Nb_{2/3})-PbTiO₃ (PMN-PT), and Pb(In_{1/2}Nb_{1/2})-Pb(Mg_{1/3}Nb_{2/3})-PbTiO₃ (PIN-PMN-PT)), and/or any sensor suitable for acoustic sensing.

Each of the optical sensors may be/include an optical resonator such as, for example, a microring resonator, a microsphere resonator, a microtoroid resonator, a microbubble resonator, a fiber-based resonator, an integrated photonic resonator, a micro-disk resonator, and/or the like. In some variations, the optical sensors may include one or more WGM optical resonators. For example, in some variations an optical sensor may be similar to any of the optical resonators described in PCT App. Nos. PCT/US2020/064094, PCT/US2021/022412, and PCT/US2021/033715. The optical sensors may include a closed loop of a transparent medium (e.g., glass, transparent polymer, silicon nitride, titanium dioxide, or any other material that is suitably optically transparent at an operation wavelength of the optical resonator) that allows some permitted frequencies of light to continuously propagate inside the closed loop, and to store optical energy of the permitted frequencies of light in the closed loop. The aforementioned is equivalent to say that the optical resonators may permit a propagation of modes (e.g., whispering gallery modes (WGMs)) traveling the concave surface of the optical resonators and corresponding to the permitted frequencies to circulate the circumference of the resonator. Each mode corresponds to propagation of a frequency of light from the permitted frequencies of light. The permitted frequencies of light and the quality factor of the optical resonators described herein may be based at least in part on geometrical parameters of the optical resonator, refractive index of the transparent medium, and refractive indices of an environment surrounding the optical resonator. Resonant frequencies (e.g., due to propagation of a set of WGMs) of the optical resonator can have high quality factors suitable for highly sensitive sensing probes. In general, the sensitivity of optical sensors can be improved by increasing the quality factor of the optical resonator. In particular, in some variations, the sensitivity can be controlled by geometrical parameters of the optical resonator. When used as ultrasound detectors, the optical resonator can have a low noise equivalent pressure and a broadband operation bandwidth. In some variations, the optical resonator may include sensing nodes formed at a cross-section of optical fibers and optical waveguides when light propagating in the optical waveguides couples in the optical fibers and propagates in circumferences of the optical fibers. In some variations the optical sensors may include integrated photonic optical resonators.

The space inside and/or around the optical resonators may be filled with an ultrasonic enhancement material, such as for example, polyvinylidene fluoride, parylene, polystyrene, and/or the like. The ultrasonic enhancement material can increase sensitivity of the optical sensors. For example, the ultrasonic enhancement material can have a relatively high elasto-optic coefficient, such that in response to the optical resonators receiving a set of ultrasound echoes, the refractive index of the ultrasonic enhancement material changes more than the refractive index of the material of a material(s) of the optical resonators (e.g., upon receiving a mechanical stress or strain induced by the set of ultrasound echoes).

The optical resonators may be coupled to the outside world to receive light, to transmit light, and to be useful in practice (e.g., for an ultrasound imaging or other sensing application in an acousto-optic system). In some implementations, the optical resonators may be operatively coupled, via an optical fiber (e.g., a tapered optical fiber), to a light source (e.g., a laser, a tunable laser, an erbium doped fiber amplifier, and/or the like) and/or a photodetector. Acousto-optic systems based on optical sensors may directly measure ultrasonic waves through the photo-elastic effect and/or physical deformation of the resonator(s) in response to the ultrasonic waves (e.g., ultrasonic echoes). Therefore, the optical sensors can be considered as optoacoustic transducers that can convert mechanical energy (e.g., acoustic energy) to optical energy. For example, in the presence of ultrasonic (or any pressure) waves, the modes traveling a resonator may undergo a spectral shift or amplitude change caused by changes in the refractive index and shape of the resonator. The spectral change can be easily monitored and analyzed in spectral domain using the photodetector. The amplitude change can also be detected by the photodetector. The photodetector eventually converts the optical energy (i.e., optical signal) propagating in the optical resonators and the optical fiber into electrical energy (i.e. electrical signal) suitable for processing with electronic circuitry. Additional spatial and other information can furthermore be derived by monitoring and analyzing optical response of optical resonators among mixed arrays. Exemplary mixed ultrasound arrays are described herein.

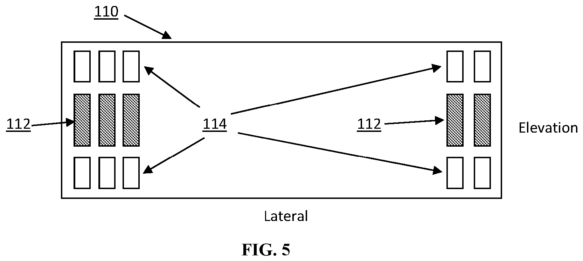

In some variations, the mixed array 110 may include one or more rows in an elevation dimension. For example, the array elements (of the first type and the second type) may be collectively positioned in a rectangular array including a number of rows and a number of columns. In some variations, as shown in FIG. 5, the mixed array 110 may include 3 rows of elements in an elevation dimension. The 3 rows include an inner row and two outer rows. The two outer rows may be made of the second type 114 (e.g., optical resonator such as a WGM optical resonator). The inner row may be made of the first type 112 (e.g., PZT transducer or another type of transducer). The two outer rows may include equal number of elements that are positioned in parallel in a corresponding column. Each pair of elements 114 positioned in the same column in the two outer rows may be optionally connected (e.g., electrically connected or electromagnetically coupled) to form a single combined outer element for a 1.25 dimensional (1.25D) array configuration or a 1.5 dimensional (1.5D) array configuration.

Although FIG. 5 depicts the mixed array 110 having three rows, in some variations, the number of rows may be any odd number such as 3, 5 ... 2*n +* 1, where *n* is an integer. In some variations, array elements of the first type 112 may be arranged in the center row of a set of an odd number of rows. For example, a 1.5D array configuration may include 5 rows with a PZT transducer row in the center row, two optical resonator rows adjacent to the center row, and two PZT transducer rows on the outermost rows adjacent to the optical resonator rows. Having the center row include transducers may be advantageous in some variations. For example, since the center row includes transducer elements of first type 112 that can perform both transmission and reception of ultrasound waves, the elevation apodization profile does not have a "dip" in the middle, for both the transducers' transmission mode and reception mode. This dip occurring in an elevation apodization profile can degrade image quality and introduce image artifacts. Accordingly, arranging transducer elements of the first type 112 in the center row (e.g., as shown in FIG. 5) may advantageously help avoid such degradation in image quality and image artifacts. In some variations, however, the mixed sensor array may include optical resonators in a center row.

In some variations, the number of rows may be any even number such as 2, 4 ... 2*n,* where *n* is an integer. For example, a 1.25D array configuration or a 1.5D array configuration may include at least two rows with a first number of PZT transducer elements (or other transducer elements) in one row and a second number of optical sensor elements in the other row. In some variations, the first and second numbers may be the same, while in other variations the first and second numbers may be different (e.g., one row may include 128 array elements, while another row may include 192 array elements).

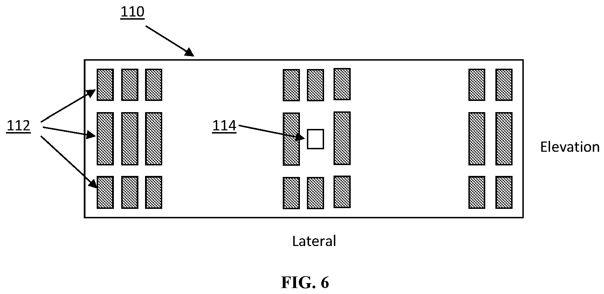

FIG. 6 is a schematic description of an exemplary mixed array. The mixed array 110 may include one or more array elements of a first type (e.g., PZT transducer or another type of transducer) and one or more array elements of a second type (e.g., optical sensor such as, for example, a WGM resonator). The mixed array 110 may include at least one row that has at least one array element of the first type and at least one array element of the second type. As shown in FIG. 6, the mixed array 110 may, for example, include a center row including at least one array element of the first type and at least one array element of the second type. For example, the center row may have a single array element of the second type, while the other rows may only have array elements of the first type. The single array element of the second type can be an optical resonator that is about equal to or smaller than a wavelength of the transmitted acoustic waves. In some variations, the use of a single optical resonator can minimize the complexity of probe manufacturing while utilizing the ultra-high sensitivity of the optical sensor for image quality improvement.

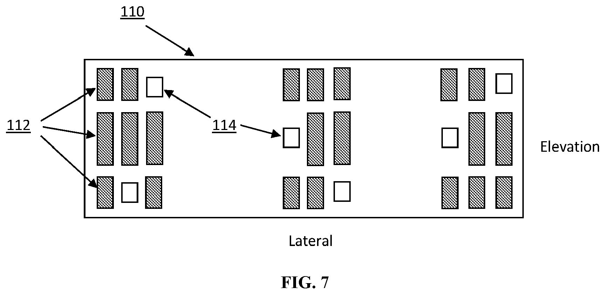

FIG. 7 is a schematic description of an exemplary mixed array. The mixed array 110 may include two or more rows. Each of the two or more rows may have at least one array element of the first type (e.g., PZT transducer or another type of transducer) and at least one array element of the second type (e.g., optical sensor such as a WGM resonator). The array elements of the second type may be spatially distributed in a regular pattern or may be spatially distributed in an irregular pattern (e.g., random pattern). A set of the elements on the inner row and two outer rows may include optical resonators 114 and the rest of the elements include the first type 112 that include, for example, a PZT transducer (s) and/or a CMUT transducer (s). In some configurations, a spatial distribution of positions of the optical resonators 114 can be random. In some configurations, a spatial distribution of positions of the optical resonators 114 can follow a dispositioning pattern (e.g., be the same, shift to the right by one cell among sensor elements, shift to down by two cells among sensor elements). A size of the optical sensor can be smaller than or the same as the size of the first type 112.

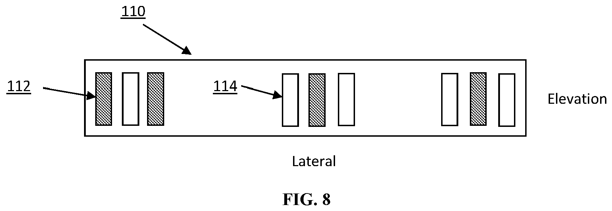

FIG. 8 is a schematic description of an exemplary 1D mixed array 110 that includes a single row including multiple array elements or sensor elements. The multiple array elements may include at least one array element of the first type 112 (e.g., PZT transducer or another type of transducer) and at least one array element of the second type 114 (e.g., optical sensor such as a WGM optical resonator). In some configurations, the spatial distribution of those of the first type 112 and those of the second type 114 may be random. In some configurations, the spatial distribution of the array elements of the first type 112 and the array elements of the second type 114 may follow a dispositioning pattern. Compared to a traditional 1D array that includes only one type of sensor, the mixed array may have an improved performance in sensing bandwidth and/or sensitivity due to the addition of the optical sensors.

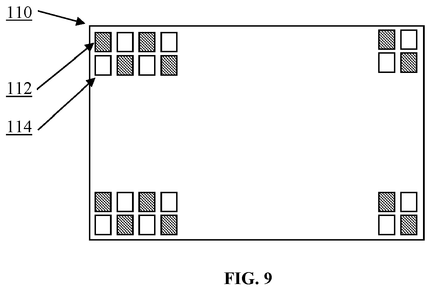

FIG. 9 is a schematic description of an exemplary 2D mixed array 110 arranged in a rectangular configuration and may include *N* × *M* sensor elements, where *N* is the number of rows and *M* is the number of columns and are both integers. In some implementations, the number of rows and/or the number of columns may be greater than 31 rows and/or 31 columns. For example, a 2D mixed array may include 64 × 96 = 6,144 sensor elements. The mixed array 110 may include one or more array elements of a first type (e.g., PZT transducer or another type of transducer) and one or more array elements of a second type (e.g., optical sensor such as a WGM optical resonator) that may be collectively positioned in a rectangular arrangement. In some configurations, the spatial distribution of the first type 112 and the second type 114 may be random. In some configurations, the spatial distribution of the first type 112 and the second type 114 may follow a dispositioning pattern.

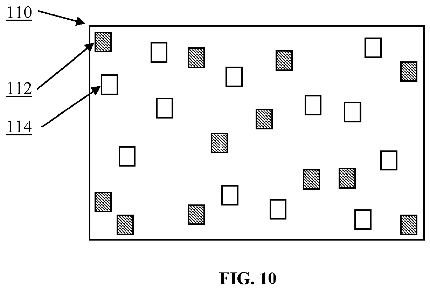

FIG. 10 is a schematic description of an exemplary 2D mixed array 110 in a sparse array configuration. Arranging the mixed array 110 in the sparse array configuration instead of a fully sampled arrangement may reduce the total number of sensor elements used to manufacture the mixed array. For example, a sparse 2D array having the same size of a fully sampled 2D, may include only 1000 sensor elements compared to the 64 × 96 = 6,144 sensor elements of in the fully sampled mixed array. The mixed array 110 may include one or more array elements of a first type (e.g., PZT transducer or another type of transducer) and one or more array elements of a second type (e.g., optical sensor such as a WGM optical resonator), collectively positioned in a sparse array configuration. The spatial distribution of array elements of the first type 112 and the array elements of the second type 114 may be random or follow a statistical distribution (e.g., a normal distribution, a Gaussian distribution, and/or the like). By using the sparse spatial distribution of array elements of the first type 112 and the second type 114, generation of grating lobes in an image produced by the mixed array may be reduced/prevented. A spatial distribution of the array elements of the first type 112 may be the same as, similar to, or different from, a spatial distribution of the array elements of the second type 114. For example, a first set of positions of a set of optical sensors in the mixed array 110 may have a uniform distribution and second set of positions of a set of PZT transducers in the mixed array 110 may have a normal distribution.

### Methods of Performing Synthetic Aperture Imaging

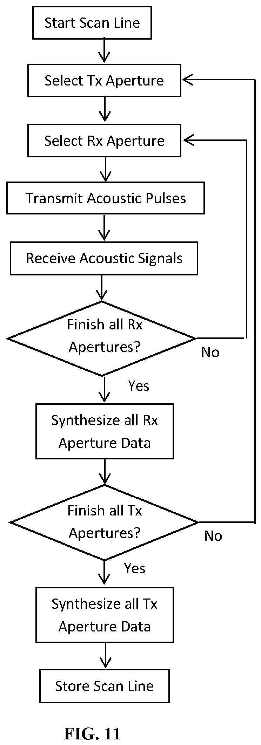

FIGS. 11-17 described below illustrate aspects of exemplary methods of performing synthetic aperture imaging. The methods of performing synthetic aperture imaging may be executed by a synthetic aperture computing device (not shown) that is part of and/or is operatively coupled to a synthetic aperture imaging system (such as the synthetic aperture imaging system 100 shown and described with respect to FIG. 1). The synthetic aperture computing device may include a set of electronic circuitry such as a processor, a memory, and a communication interface. The processor can include, for example, a hardware based integrated circuit (IC) or any other suitable device to run or execute a set of instructions/codes. For example, the processor can include a general purpose processor, a central processing unit (CPU), an accelerated processing unit (APU), an application specific integrated circuit (ASIC), a microprocessor, a field programmable gate array (FPGA) chip, a graphics processing unit (GPU), a digital signal processing (DSP) chip, and/or the like. The memory can store, for example, code that includes instructions to cause the processor to perform one or more processes or functions (e.g., filtering signals, amplifying signals, phase matching, noise reduction, selecting apertures, and/or the like). The memory may be/include, for example, a memory buffer, a random access memory (RAM), a read-only memory (ROM), a flash drive, a secure digital (SD) memory card, and/or the like. The communication interface can be/include a USB interface, a PCIe interface, or a hardware component that is operatively coupled to the processor and/or the memory and may enable communication of the synthetic aperture computing device with components of the synthetic aperture imaging system and/or in some variation, external device and/or network of devices (e.g., the Internet).

The synthetic aperture computing device may include an application as a software stored in the memory and executed by the processor. For example, the application can include code to cause the processor to select aperture, analyze signals, generate an image, and/or the like. Alternatively, the application can be implemented on a hardware-based device. For example, the application can include a digital circuit(s) or an analog circuit(s) that can cause the synthetic aperture computing device to filter signals, amplify signals, and/or delay signals.

FIG. 11 is a flowchart of an exemplary method of performing synthetic aperture imaging using a mixed array configured to generate each ultrasound image frame on a serial scan line-by-scan line basis. The synthetic aperture imaging system can begin performing synthetic aperture imaging after receiving an indication signal to start a new scan line. The synthetic aperture imaging system may then select a transmit aperture(s) that includes one or more array elements of a first type (e.g., a PZT transducer(s)). The synthetic aperture imaging system may then connect transmit channels of a transmit beamformer (such as the transmit beamformer 146 as shown and described with respect to FIG. 2) to selected array elements of the first type. The synthetic aperture imaging system then selects a receive aperture(s) that includes one or more array elements of a second type (e.g., optical sensor(s)). In some variations, the receive aperture(s) may further include the one or more array elements of the first type. Generally, selection of the receive apertures can be more complicated than selection of the transmit apertures because there are at least three possible types of receive apertures: receive apertures with solely array elements of the first type, receive apertures with solely array elements of the second type, or receive apertures with mixed array elements of the first type and the second type.

Once the transmit and receive apertures are selected and connected to the system channels, a front-end (such as the front-end 140 shown and described with respect to FIG. 1) of the synthetic aperture imaging system sends out electric signals to excite array elements of the transmit aperture to generate acoustic signals (e.g., pulses) and transmit the acoustic signals towards a target of imaging. A receive aperture then receives acoustic echoes in response to those acoustic signals, generates signals (e.g., electrical signals) corresponding to the acoustic echoes, and transmits the signals to a receive beamformer of the front-end. If the synthetic aperture imaging system includes more than one receive aperture for the same transmit aperture, the next receive aperture(s) will be selected in order to obtain additional acoustic echoes. When all receive apertures get selected at least once and corresponding signals are acquired, the receive beamformer may synthesize (e.g., coherently combine, phase match, frequency match, amplitude match, sum, and/or the like) the signals generated from all the receive apertures. Subsequently, the system may repeat cycling through all of the receive apertures for each transmit aperture. When all transmit apertures get selected at least once, the synthetic aperture imaging system will synthesize signals generated from all the transmit apertures to produce a complete, synthesized aperture including all the receive apertures and transmit apertures. The above-described process may be performed for multiple scan lines for each frame of ultrasound imaging. The synthetic aperture imaging system can then store each frame in memory and/or transmit the frame on a display included or operatively coupled to the synthetic imagine system. The above-described process may be performed for multiple frames of ultrasound imaging.

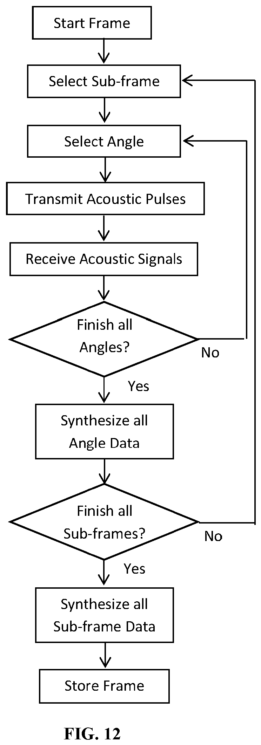

FIG. 12 is a flowchart of an exemplary method of performing synthetic aperture imaging using a mixed array configured to generate each ultrasound image frame from multiple scan lines in parallel processing. The synthetic aperture imaging system can begin performing synthetic aperture imaging after receiving an indication signal to start a new frame. The synthetic aperture imaging system may then select a first sub-frame of a complete frame. The sub-frame may include, for example, a subset of scan lines forming the complete image frame (e.g., 32 scan lines). The synthetic aperture imaging system may then select a transmit aperture, a receive aperture, and a steering angle. The selected receive aperture can be made solely of array elements of the first type, solely array elements of the second type, or mixed array elements of the first type and the second type. Once appropriate transmit and receive apertures for the sub-frame and steering angle are selected and connected to the system channels, a front-end (such as the front-end 140 shown and described with respect to FIG. 1) of the synthetic aperture imaging system sends out electric pulses to excite array elements of the transmit apertures to generate acoustic signals (e.g., pulses) and transmit the acoustic signals towards a target of imaging through the selected transmit aperture(s). The selected receive aperture(s) then receive acoustic echoes in response to those acoustic signals, generate signals (e.g., electrical signals and optical signals) corresponding to the acoustic echoes, and transmit the signals to a receive beamformer of the front-end.

If the synthetic aperture imaging system is required to select multiple transmit angles for the sub-frame, additional steering angles may be selected in order to obtain additional acoustic echoes, and the above-described process may be repeated for each additional steering angle for the sub-frame. When all steering angles for the sub-frame get selected at least once and corresponding signals are acquired, the receive beamformer may coherently synthesize (e.g., coherently combine, phase match, frequency match, amplitude match, sum, and/or the like) the signals generated from all steering angles for the sub-frame. Subsequently, the system may repeat cycling through all of the steering angles for each sub-frame. When all sub-frames get selected at least once, the synthetic aperture imaging system may synthesize (e.g., coherently combine, phase match, frequency match, amplitude match sum, and/or the like) the signals generated from all sub-frames to generate a complete frame. The synthetic aperture imaging system can then store the frame in the memory and/or transmit the frame to a display included or operatively coupled to the synthetic aperture imaging system. The above-described process may be performed for multiple frames of ultrasound imaging.

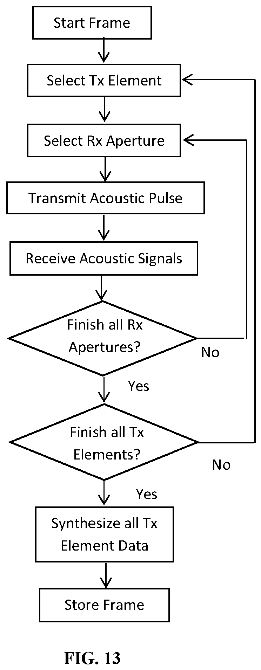

FIG. 13 is a flowchart of an exemplary method of performing synthetic aperture imaging using a mixed array configured to generate each ultrasound image frame from multiple receive apertures per transmit element (e.g., transducers of the first type, such as a PZT transducer or another type of transducer) intended to operate for imaging. The synthetic aperture imaging system can begin performing synthetic aperture imaging after receiving an indication signal to start a new frame. The synthetic aperture imaging system may then select a first transmit element by connecting the transmit channel of a transmit beamformer (such as the transmit beamformer 146 as shown and described with respect to FIG. 2) to the first transmit element. The first transmit element is an array element of the first type that is capable of generating acoustic signals. The synthetic aperture imaging system then selects a receive aperture(s) that includes one or more array elements of the second type and may further include the one or more array elements of the first type. There are three possible types of receive apertures: receive apertures with solely array elements of the first type, receive apertures with solely array elements of the second type, or receive apertures with array elements of both the first type and the second type.

Once the transmit element and the receive apertures are selected and connected to the system channels, the front-end transmits electric signals to excite the transmit element and to generate acoustic signals and transmit the acoustic signals towards a target of imaging. A receive aperture then receives acoustic echoes in response to those acoustic signals, generates signals corresponding to the acoustic echoes, and transmits the signals to a receive beamformer of the front-end. If the synthetic aperture imaging system includes more than one receive aperture for the same transmit element, additional receive apertures will be selected in order to obtain additional acoustic echoes associated with transmission from that transmit element. When all receive apertures get selected at least once and corresponding signals are acquired, the receive beamformer may synthesize (e.g., coherently combine, phase match, frequency match, amplitude match, sum, and/or the like) the signals generated from all the receive apertures for that transmit element. Subsequently, the system may repeat cycling through all of the transmit elements used in the imaging. When all transmit elements get selected, the synthetic aperture imaging system may synthesize all the transmit elements to produce a synthesized aperture to generate a single frame or multiple frames. The synthetic aperture imaging system can then store the frame(s) in memory and/or transmit the frame(s) to a display included or operatively coupled to the synthetic imagine system. The above-described process may be performed for continuously scanning of a patient.

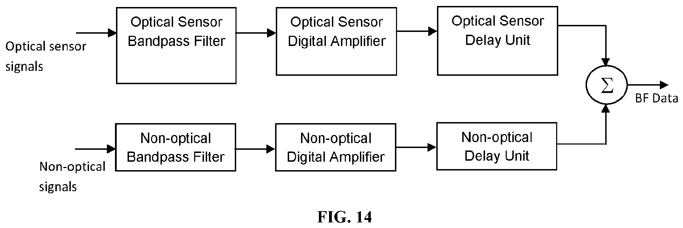

FIG. 14 is a block diagram of an exemplary method of synthesizing acoustic data obtained using a mixed array, according to some variations. As described herein, the mixed array includes one or more array elements of a first type (e.g., non-optical transducers) and one or more array elements of a second type (e.g., optical sensors such as WGM optical resonators). Therefore, the mixed array generates optical signals and non-optical signals. The optical sensor signals and non-optical sensor signals may have different signal paths. Each of the optical sensors and the non-optical sensors has a different physical location in the mixed array, and optical resonators generally have different frequency responses, sensitivities, and amplitudes compared to non-optical sensors. As a result, signals from optical resonators may require processing through different filters (e.g., low-pass filters, band-pass filters, high-pass filters, digital filters, and/or the like), amplifiers (e.g., digital amplifiers), and/or phase delays to compensate for their differences relative to signals from non-optical sensors, before the optical sensor signals and the non-optical sensor signals may be effectively combined by a receive beamformer.

For example, as shown in FIG. 14, different bandpass filters can shape the waveforms of the received optical sensor signals and the non-optical sensor signals to improve detail resolution and signal-to-noise ratio (SNR). At least one optical sensor bandpass filter may be used to shape the received optical sensor signals, and at least one non-optical bandpass filter may be used to shape the received non-optical sensor signals (e.g., such that the optical sensor signals and the non-optical sensor signals are matching in frequency). The optical sensor bandpass filter and the non-optical bandpass filter may have different characteristics to account for differences in frequency response of the optical resonators and non-optical sensors. As ultrasound signals propagate in soft tissues, waveforms or spectral shapes of the ultrasound signals may vary with penetration depth. To take such variation in waveforms and spectral shapes into account, the synthetic aperture imaging system may choose filters based on penetration depth, the waveforms, and/or spectral shapes.

In addition, different amplifiers can also provide gain values and/or apodization profiles to the received optical sensor signals and non-optical sensor signals to produce optimal or near-optimal beam patterns with minimum or near-minimum side lobes. For example, at least one optical sensor digital amplifier may be used to provide a suitable gain and/or apodization profile associated with the optical sensor signals, and at least one non-optical digital amplifier may be used to provide a suitable gain and/or apodization profile associated with the non-optical sensor signals (e.g., such that the optical sensor signals and the non-optical sensor signals are matching in amplitude). The gain and/or apodization profile provided by the optical sensor digital amplifier may be different than those applied by the non-optical digital amplifier to account for different sensitivities of the optical resonator and non-optical sensors. The gains and/or the apodization profiles can include preset and/or predetermined values stored in a memory of the synthetic aperture imaging system. In some instances, the synthetic aperture imaging system can be configured to generate the gains and/or the apodization profiles dynamically. In some instances, the gains and/or the apodization profiles of the amplifiers can be a constant number or can be variable as a function of depth.

In addition, different phase delays may be applied to the optical sensor signals and the non-optical sensor signals based on positions and/or position differences between optical resonators and/or non-optical sensors. An optical sensor delay unit may apply a suitable phase delay to the optical sensor signals, and a non-optical delay unit may apply a suitable phase delay on the non-optical sensor signals (e.g., such that the optical sensor signals and non-optical sensor signals are matching in phase). The phase delays applied by the optical sensor delay unit and the non-optical delay unit may be different to account for the different positions of the optical resonators and the non-optical sensors. The phase delays can include preset/predetermined values stored in the memory. In some instances, the synthetic aperture imaging system can be configured to generate the phase delays (e.g., a phase delay profile) dynamically. In some instances, the phase delay may also account for other factors. For example, the phase delay may incorporate a stored delay value based on a nominal or known acoustic lens thickness, and/or a dynamic stored delay value determined using an adaptive system configured to detect phase aberrations and/or other imperfections of the acoustic lens and/or the media. Beside the lens, both optical sensors and non-optical transducers may include other layers (e.g., a matching layer, a coating layer, and/or the like) between the sensor surface and the patient body. In addition to thickness consideration, acoustic velocity can be another parameter in determining a final delay profile(s) for synthetic aperture beamforming.

After application of filters, amplifiers, and phase delays as described above to process the received optical sensor signals and non-optical sensor signals, the optical sensor signals and non-optical sensor signals may be combined and communicated to the receive beamformer to form an image. In some variations, a combination of the optical sensor signals and non-optical sensor signals may be a coherent combination.

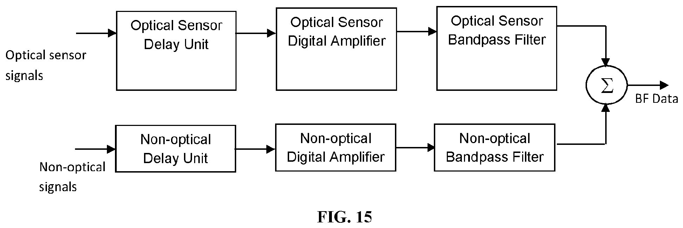

Although FIG. 14 illustrates a particular sequence of signal processing (filtering, then amplifying, then applying a phase delay), it should be understood that in some variations the above-described signal processing steps may be performed in any suitable order. For example, FIG. 15 is a block diagram of an exemplary method of synthesizing acoustic data obtained using a mixed array, according to some variations. As shown in FIG. 15, instead of applying the phase delays to the optical sensor signals and non-optical sensor signals after filtering and amplifying, the synthetic aperture imaging system may apply the phase delays first, followed by amplifying and then filtering. As another example, in some variations, the optical sensor signals and non-optical sensor signals may be processed by applying the phase delays, then performing filtering, and then performing amplification. In other words, synthesizing the optical sensor signals with the non-optical sensor signals from a mixed array may include any permutation of filtering, amplifying, and applying a phase delay.

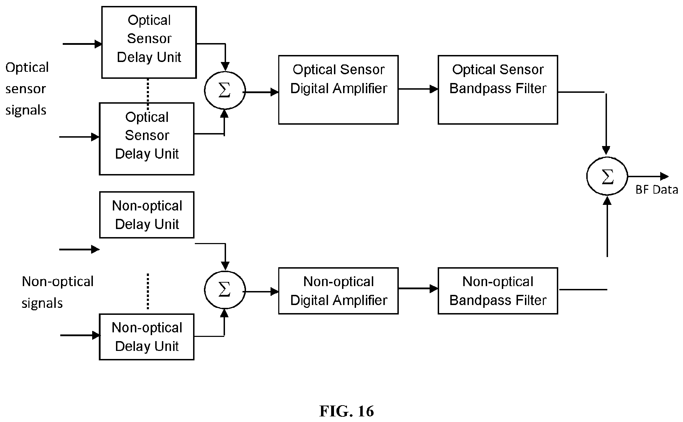

FIG. 16 is a block diagram of an exemplary method of synthesizing acoustic data obtained using a mixed array, according to some variations. The synthetic aperture imaging system may be configured to apply a first set of phase delays to the optical sensor signals using multiple respective optical sensor delay units (e.g., for each optical sensor signal) in order to phase match all of the received optical sensor signals. The resulting phase matched optical sensor signals may then be combined together. Similarly, the synthetic aperture imaging system may be configured to apply a second set of phase delays to the non-optical sensor signals using multiple respective non-optical delay units (e.g., for each non-optical signal) in order to phase match all of the received non-optical sensor signals. The resulting phase matched non-optical sensor signals may then be combined together. The synthetic aperture imaging system may be configured to further apply amplifier(s) and filter(s) to each of the combined optical sensor signals and the combined non-optical sensor signals, similar to that described above with respect to FIG. 14. For example as shown in FIG. 16, the combined optical sensor signals may be further processed with at least one optical sensor digital amplifier and at least one optical sensor bandpass filter (in either order), and the combined non-optical sensor signals may be further processed with at least one non-optical digital amplifier and at least one non-optical bandpass filter (in either order), such that the combined optical sensor signals and the combined non-optical sensor signals are matching in amplitude and frequency response. The phase matched, amplitude matched, and/or frequency matched optical sensor signals and non-optical sensor signals may be combined and communicated to the receive beamformer to form an image. Compared to the variations shown and described with respect to FIGS. 14 and 15, the variation of FIG. 16 may have an advantage in reducing numbers of filters and amplifiers used in the synthetic aperture imaging system, thereby reducing manufacturing costs, etc.

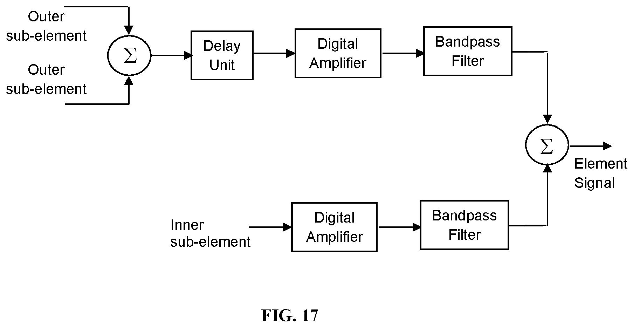

FIG. 17 is a block diagram of an exemplary method of synthesizing acoustic data obtained using a mixed array, according to some variations. The synthetic aperture imaging system may be configured to synthesize a single element including an inner sub-element and two outer sub-elements (such as the 1.5D array shown and described with respect to FIG. 5). The two outer sub-elements may have the same size and be positioned on each side of the inner sub-element. Therefore, signals originating from the two outer sub-elements may be combined (e.g., summed) together before applying delays (for elevation focusing) to those signals. After combining signals from the two outer-elements, a phase delay, an amplifier and/or a filter can be applied to the combined signal in any suitable order. Additionally, a signal originating from the inner sub-element can be separately amplified and filtered and finally combined with the combined signal of the two outer sub-elements. Once the inner sub-element and outer sub-elements for each element are synthesized as described above, a 1.5D array or a 2D array can be simplified to a 1D linear array for beamformation. As a result, the number of phase delay processes can be significantly reduced. Although FIG. 17 depicts three sub-elements (one inner sub-element and two outer sub-elements), it should be understood that the process described above may be applied to a system including more than three sub-elements. For example, in some instances, the process described above may be applied to a 1.5D array with two or more than three sub-elements. For example, the element can include five sub-elements, seven sub-elements, and/or the like.

In some variations, the approach described with respect to FIG. 17 can be also extended to more than one element. For instance, two adjacent elements of a 1.5D array can be synthesized to be regarded as a single element whose pitch is equivalent to a sum of pitches for the two adjacent elements. The resulting 1D array generated by such approach has only half the original number of elements. More generally, *n* adjacent elements can be synthesized to form a larger element and hence reduce number of effective elements for synthetic aperture by *n* times, where *n* is an integer greater than 1.

In some variations, an elevation beamformation is performed before a lateral beamformation. In some variations, however, order of beamformations may be reversed. That is to say, the lateral beamformation may be performed before the elevation beamformation.

### Examples

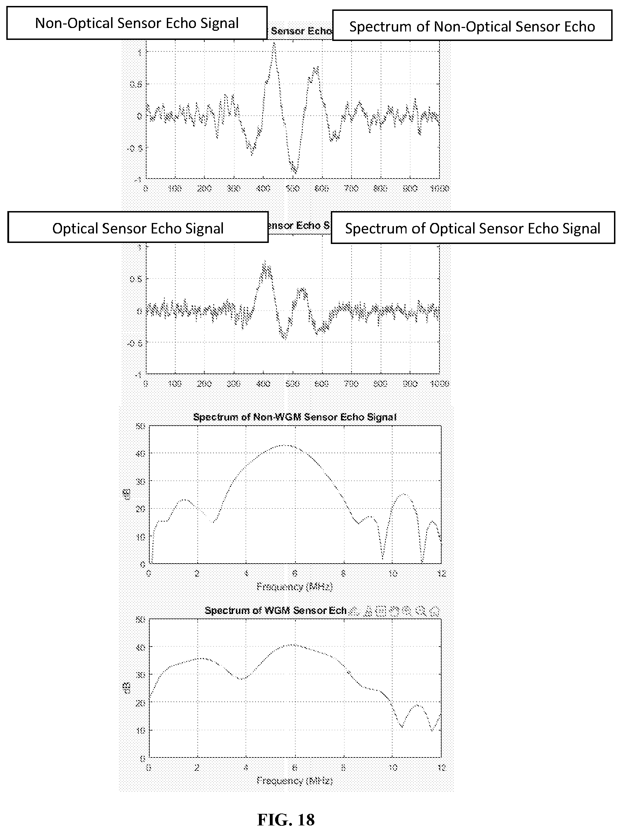

FIG. 18 shows exemplary signals generated by two types of sensors in a mixed array. The upper left corner diagram ("Non-Optical Sensor Echo Signal") shows a signal generated by a non-optical sensor in time domain and the lower left corner diagram ("Optical Sensor Echo Signal") shows a signal generated by an optical resonator in time domain. The upper right corner diagram ("Spectrum of Non-optical Sensor Echo Signal") shows a signal generated by a non-optical sensor in frequency domain and the lower right corner diagram ("Spectrum of Optical Sensor Echo Signal") shows a signal generated by an optical resonator in frequency domain. As shown, signals generated by the optical resonator are different in amplitude, frequency, phase, and noise level compared to signals generated by the non-optical sensor. Such variations in amplitude, frequency, phase, and noise level can be compensated by applying amplifiers, filters, phase delays, and noise filters to the signals such as that as described above with respect to FIGS. 14-17.

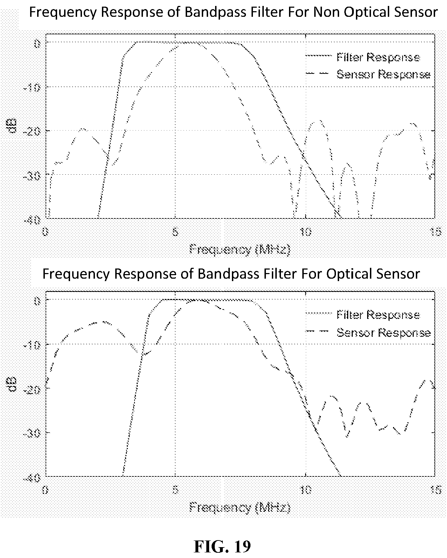

FIG. 19 shows exemplary frequency response of signals generated by non-optical sensors and optical resonators in a mixed array, and a bandpass filter frequency response appropriate for each frequency response when synthesizing non-optical sensor signals and optical sensor signals. Specifically, the dashed lines indicate the signals generated by the sensors in frequency domain (similar to that shown in FIG. 18). Additionally, the solid lines show frequency responses of two Butterworth band-pass filters designed for processing the signals generated by the optical resonator (bottom figure) and the signals generated by the non-optical sensors (top figure). As shown in FIG. 19, the optical sensor bandpass filter and the non-optical bandpass filters are determined to have different center frequencies and bandwidths each corresponding to the respective spectral response (dashed line) generated by the non-optical and the optical resonator. For example, as shown in the bottom plot of FIG. 19, the spectrum of the optical resonator has strong low frequency components between about 0 and about 4 MHz that may degrade the detail resolution of the final ultrasound image. Therefore, the band-pass filter for the optical sensor signals is designed to attenuate these low frequency components below about 4 MHz, thereby isolating the more valuable frequency components of the optical sensor signals for imaging purposes. However, when processing the non-optical sensor signals, such a 4 MHz cutoff frequency for a bandpass filter is too high to preserve the useful frequency components between 3 and 4 MHz. Therefore, the band-pass filter for the non-optical sensor signals may be designed with a lower cutoff frequency (e.g., about 3 MHz) compared to that of the bandpass filter for the optical resonator.

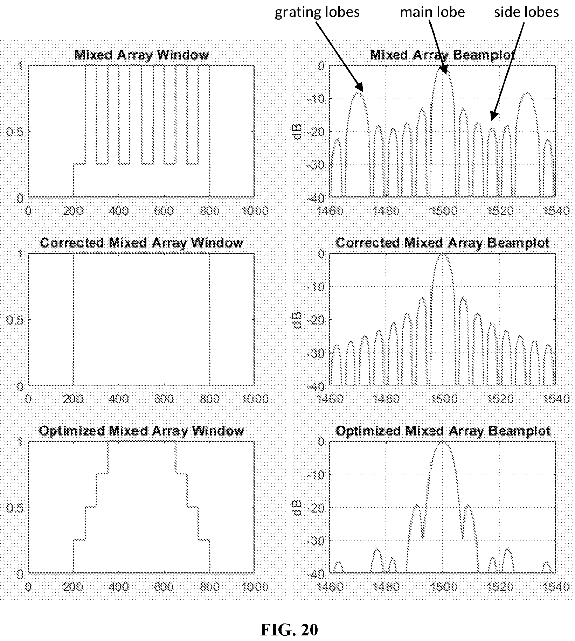

FIG. 20 shows exemplary mixed array windows and their corresponding beamplots. A beamplot is a 1D beam pattern at a certain depth in the imaging plane. A beamplot usually consists of a main lobe in the middle and side lobes with lower peak values on both sides of the main lobe. A width of the main lobe determines a spatial resolution of an ultrasound image. A level of the side lobes may determine a contrast resolution. In some instances, when element pitches are too large or element sensitivity profile are periodically uneven, grating lobes may appear on a beamplot. The grating lobes may produce undesired image artifacts including ghost images.

Three aperture window functions and their corresponding beamplots are show in FIG. 20. The top left diagram ("Mixed Array Window") presents a window function generated by the mixed array configuration shown and described with respect to FIG. 8. The unevenness in the window function is caused by a difference in sensitivity between the optical resonators and non-optical sensors in the mixed array. As shown in the top right diagram ("Mixed Array Beamplot"), the periodically uneven window produces two grating lobes with about -8.4 dB amplitude. The middle diagrams ("Corrected Mixed Array Window" and "Corrected Mixed Array Beamplot") illustrate that the grating lobes can be overcome by applying different digital amplification gains to the two types of sensors. Applying such digital amplification gains can generate a uniform window function. A similar approach may be utilized to generate a Gaussian-like apodization window to reduce the side lobes of the beamplot, as shown in the two bottom diagrams ("Optimized Mixed Array Window" and "Optimized Mixed Array Beamplot"). All three beamformer architectures presented in FIGS. 14 -16 may produce a uniform window function. However, in some instances, only beamformer architectures of FIGS. 14 and 15 may produce an arbitrary window function.

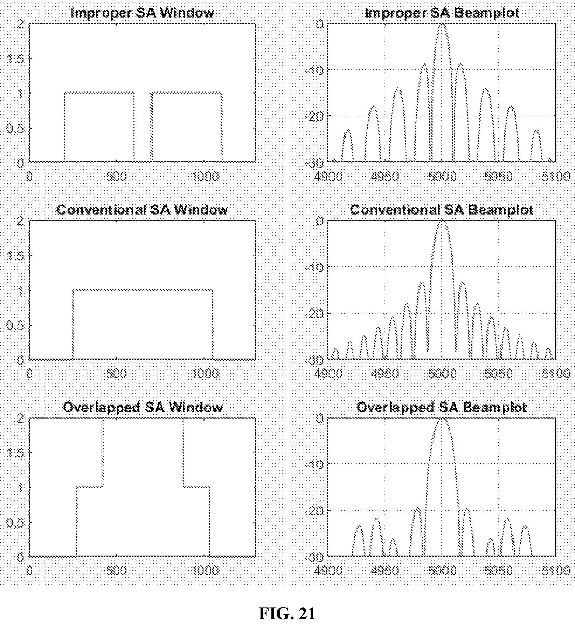

FIG. 21 shows exemplary synthesized aperture windows for mixed arrays and their corresponding beamplots. Three exemplary synthetic aperture (SA) window functions and their corresponding beamplots are shown. The top two diagrams demonstrate why two or more sub-apertures must be synthesized properly to generate a good beam pattern. The top left picture ("Improper SA window") shows an improperly synthesized aperture window function. The top right beamplot ("Improper SA Beamplot") shows elevated side lobes generated because of a gap between two sub-apertures of the synthesized aperture window function. The middle two diagrams ("Conventional SA Window" and "Conventional SA Beamplot") show a conventionally synthesized aperture with the same two sub-apertures used for the synthesized aperture in the top left picture and corresponding beamplot. The side lobes are significantly reduced than that of the beamplot in the top right. The side lobes can be further reduced by synthesizing two overlapped sub-apertures as shown in the two bottom diagrams. The synthesized aperture has a rough apodization window as shown in the bottom left picture ("Overlapped SA Window"). The resulting beamplot ("Overlapped SA Beamplot") in the right bottom diagram shows reduced side lobes due to the overlapped sub-apertures.

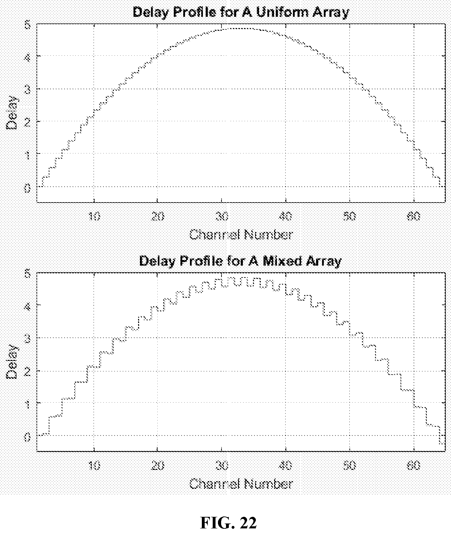

FIG. 22 shows an exemplary delay profile for a uniform array and an exemplary delay profile for a mixed array. In some variations, acoustic waves and/or signals may travel through different signal paths before the signals being summed by a beamformer. For instance, for a PZT sensor element, acoustic waves and corresponding signals may travel through acoustic lens and one or more matching layers before reaching the PZT sensor. On the other hand, for an optical resonator, acoustic waves and corresponding signals may travel through acoustic lens with different thickness and/or acoustic velocities and polymer layers with different thickness and/or acoustic velocities before reaching the optical resonator. The difference in signal paths may result in additional delays. Such extra delays introduce phase errors in beamforming and consequently degrade imaging performance including detail resolution, contrast resolution, and signal-to-noise ratio (SNR). Therefore, extra delays between the two sensor channels may be adjusted accordingly as shown in FIG. 22 and described further below.

The top plot ("Delay Profile for a Uniform Array") shows a delay profile for an aperture with the 64 elements of the same sensor. The bottom delay profile ("Delay Profile for a Mixed Array") is for an aperture with the 64 elements of two different sensors such as the mixed array shown and described with respect to in FIG. 8. An additional fixed phase delay may be added to either of the non-optical channels or the optical resonator channels to compensate for the difference in signal paths between the two types of sensors. The delay profiles shown can be used for both transmit and receive beamforming.

Although synthetic aperture imaging methods and systems for mixed arrays has been described in the context of ultrasound imaging, in some variations, the synthetic aperture imaging methods and systems can be used in application other than ultrasound imaging. For example, in some instances, the synthetic aperture imaging methods and systems can be used in metrology, signal processing, particle physics, remote sensing, aerospace applications, and/or the like.

## Claims

1. A method of acousto-optic imaging comprising:
transmitting acoustic waves;
receiving a first signal from a first sub-aperture of a sensor array; the first sub-aperture comprises one or more array elements of a first type (112);
receiving a second signal from a second sub-aperture of the sensor array; the second sub-aperture comprises one or more array elements of a second type (114) different from the first type; the second type is an optoacoustic transducer; the array elements of the first and second types configured to detect acoustic echoes corresponding to the transmitted acoustic waves; and,
combining, using a receive beamformer, the first signal and the second signal to obtain a combined signal for forming an image; the combined signal is from a synthesized aperture comprising the first sub-aperture and the second sub-aperture of the sensor array.

2. The method of claim 1, further comprising: phase matching the first signal and the second signal.

3. The method of claim 2, wherein phase matching the first signal and the second signal comprises applying a first delay to the first signal or a second delay to the second signal, the first delay and the second delay being determined based at least in part on a difference between a first propagation time from the one or more array elements of the first type (112) to a medium being imaged and a second propagation time from the one or more array elements of the second type (114) to the medium.

4. The method of claim 3, wherein the first delay or the second delay is determined based at least in part on a thickness and acoustic velocity of an acoustic lens, or a thickness and acoustic velocity of an acoustic matching layer, or a thickness and acoustic velocity of each of an acoustic lens and an acoustic matching layer.

5. The method of claim 1, further comprising any of:
a) filtering the first signal to reduce noise in the first signal and filtering the second signal to reduce noise in the second signal;
b) amplifying the first signal or the second signal by an amplification gain to amplitude match the first signal and the second signal.

6. The method of claim 2, wherein the first signal is a combination of signals originating from a plurality of array elements of the first type or the second signal is a combination of signals originating from a plurality of array elements of the second type, or both.

7. The method of claim 6, further comprising one or more of the following, before phase matching the first signal and the second signal: generating the first signal by combining signals originating from a plurality of array elements of the first type (112), or a plurality of array elements of the first type and the second type (114); and generating the second signal by combining signals originating from a plurality of array elements of the second type, or a plurality of array elements of the first type and the second type.

8. The method of claim 7, further comprising any of:
a) forming a larger effective array element from a plurality of array elements of the first type (112), the second type (114), or both the first and second types;
b) reducing a dimensionality of the synthesized aperture.

9. The method of claim 1, further comprising: frequency matching the first signal and the second signal; after frequency matching the first signal and the second signal, amplitude matching the first signal and the second signal; and after frequency matching and amplitude matching the first signal and the second signal, phase matching the first signal and the second signal.

10. The method of claim 1, wherein the combination of the first signal and the second signal is a coherent combination.

11. The method of claim 1, further comprising: selecting the first sub-aperture for transmitting acoustic signals; and selecting the first sub-aperture or the second sub-aperture for receiving acoustic echoes in response to the acoustic signals.

12. An apparatus for imaging a target, comprising:
a transmitter for transmitting acoustic waves;
one or more array elements of a first type (112) forming a first sub-aperture;
one or more array elements of a second type (114) different from the first type and forming a second sub-aperture, the second type being an optoacoustic transducer;
the array elements of the first and second types configured to detect acoustic echoes corresponding to the transmitted acoustic waves;
the first sub-aperture receives a first signal having a first phase and the second sub-aperture receives a second signal having a second phase; and,
a front-end comprising a receive beamformer and configured to obtain a combined signal for forming an image; the combined signal is from a synthesized aperture comprising the first sub-aperture and the second sub-aperture.

13. The apparatus of claim 12, the receive beamformer configured to form the image by phase matching the first signal and the second signal.

14. The apparatus of claim 13, wherein phase matching the first signal and the second signal comprise applying a first delay to the first signal or a second delay to the second signal, the first delay and the second delay being determined based at least in part on a difference between a first propagation time from the one or more array elements of the first type (112) to a medium being imaged and a second propagation time from the one or more array elements of the second type (114) to the medium.

15. The apparatus of claim 14, wherein the first delay or the second delay is determined based at least in part on a thickness and acoustic velocity of an acoustic lens, or a thickness and acoustic velocity of an acoustic matching layer, or a thickness and acoustic velocity of each of an acoustic lens and an acoustic matching layer.

16. The apparatus of claim 12, wherein the receive beamformer configured to form the image by any of:
a) filtering the first signal to reduce noise in the first signal and filtering the second signal to reduce noise in the second signal;
b) amplifying the first signal or the second signal by an amplification gain to amplitude match the first signal and the second signal;
c) frequency matching the first signal and the second signal; after frequency matching the first signal and the second signal, amplitude matching the first signal and the second signal; and after frequency matching and amplitude matching the first signal and the second signal, phase matching the first signal and the second signal.

17. The apparatus of claim 12, wherein the first signal is a combination of signals originating from a plurality of array elements of the first type (112) or the second signal is a combination of signals originating from a plurality of array elements of the second type (114).

18. The apparatus of claim 12, wherein the combination of the first signal and the second signal is a coherent combination.

19. The apparatus of claim 12, wherein the front-end is further configured to combine the first signal and the second signal by any of:
a) selecting the first sub-aperture for transmitting acoustic signals; and selecting the first sub-aperture or the second sub-aperture for receiving acoustic echoes in response to the acoustic signals;
b) selecting an element from the one or more array elements of the first type (112) for transmitting acoustic signals; and selecting the first aperture or the second sub-aperture for receiving acoustic echoes in response to the acoustic signals.

## Patentansprüche

1. Verfahren zur akustooptischen Bildgebung, umfassend:
Übertragen von Schallwellen;
Empfangen eines ersten Signals von einer ersten Teilapertur eines Sensorarrays; die erste Teilapertur umfasst ein oder mehrere Array-Elemente eines ersten Typs (112);
Empfangen eines zweiten Signals von einer zweiten Teilapertur des Sensorarrays; die zweite Teilapertur umfasst ein oder mehrere Arrayelemente eines zweiten Typs (114), die sich vom ersten Typ unterscheiden; der zweite Typ ist ein optoakustischer Wandler; die Arrayelemente des ersten und zweiten Typs sind so konfiguriert, dass sie akustische Echos erfassen, die den übertragenen akustischen Wellen entsprechen; und,
Kombinieren des ersten und des zweiten Signals mittels eines Empfangs-Beamformers zu einem kombinierten Signal zur Bilderzeugung; das kombinierte Signal stammt von einer synthetisierten Apertur, die die erste und die zweite Teilapertur des Sensorarrays umfasst.

2. Verfahren nach Anspruch 1, ferner umfassend: Phasenanpassung des ersten Signals und des zweiten Signals.

3. Verfahren nach Anspruch 2, wobei die Phasenanpassung des ersten Signals und des zweiten Signals das Anwenden einer ersten Verzögerung auf das erste Signal bzw. einer zweiten Verzögerung auf das zweite Signal umfasst, wobei die erste und die zweite Verzögerung zumindest teilweise auf der Grundlage einer Differenz zwischen einer ersten Laufzeit von einem oder mehreren Array-Elementen des ersten Typs (112) zu einem abzubildenden Medium und einer zweiten Laufzeit von einem oder mehreren Array-Elementen des zweiten Typs (114) zu dem Medium bestimmt werden.

4. Verfahren nach Anspruch 3, wobei die erste oder die zweite Verzögerung zumindest teilweise auf der Grundlage der Dicke und der Schallgeschwindigkeit einer akustischen Linse oder der Dicke und der Schallgeschwindigkeit einer akustischen Anpassungsschicht oder der Dicke und der Schallgeschwindigkeit sowohl einer akustischen Linse als auch einer akustischen Anpassungsschicht bestimmt wird.

5. Verfahren nach Anspruch 1, ferner umfassend:
a) Filtern des ersten Signals zur Rauschunterdrückung und Filtern des zweiten Signals zur Rauschunterdrückung;
b) Verstärken des ersten oder zweiten Signals, um die Amplituden beider Signale anzugleichen.

6. Verfahren nach Anspruch 2, wobei das erste Signal eine Kombination von Signalen ist, die von einer Vielzahl von Array-Elementen des ersten Typs stammen, oder das zweite Signal eine Kombination von Signalen ist, die von einer Vielzahl von Array-Elementen des zweiten Typs stammen, oder beides.

7. Verfahren nach Anspruch 6, ferner umfassend einen oder mehrere der folgenden Schritte vor der Phasenanpassung des ersten Signals und des zweiten Signals: Erzeugen des ersten Signals durch Kombinieren von Signalen, die von einer Vielzahl von Array-Elementen des ersten Typs (112) oder einer Vielzahl von Array-Elementen des ersten und des zweiten Typs (114) stammen; und Erzeugen des zweiten Signals durch Kombinieren von Signalen, die von einer Vielzahl von Array-Elementen des zweiten Typs oder einer Vielzahl von Array-Elementen des ersten und des zweiten Typs stammen.

8. Verfahren nach Anspruch 7, ferner umfassend:
a) Bilden eines größeren effektiven Array-Elements aus mehreren Array-Elementen des ersten Typs (112), des zweiten Typs (114) oder beider Typen;
b) Reduzieren der Dimensionalität der synthetisierten Apertur.

9. Verfahren nach Anspruch 1, ferner umfassend: Frequenzanpassung des ersten Signals und des zweiten Signals; nach der Frequenzanpassung des ersten Signals und des zweiten Signals, Amplitudenanpassung des ersten Signals und des zweiten Signals; und nach der Frequenz-und Amplitudenanpassung des ersten Signals und des zweiten Signals, Phasenanpassung des ersten Signals und des zweiten Signals.

10. Verfahren nach Anspruch 1, wobei es sich bei der Kombination des ersten Signals und des zweiten Signals um eine kohärente Kombination handelt.

11. Verfahren nach Anspruch 1, ferner umfassend: Auswählen der ersten Teilapertur zum Übertragen akustischer Signale; und Auswählen der ersten oder der zweiten Teilapertur zum Empfangen akustischer Echos als Reaktion auf die akustischen Signale.

12. Vorrichtung zur Abbildung eines Ziels, umfassend:
einen Sender zum Übertragen von Schallwellen;
ein oder mehrere Array-Elemente eines ersten Typs (112), die eine erste Teilöffnung bilden;
ein oder mehrere Array-Elemente eines zweiten Typs (114), die sich vom ersten Typ unterscheiden und eine zweite Teilöffnung bilden, wobei es sich bei dem zweiten Typ um einen optoakustischen Wandler handelt;
die Array-Elemente des ersten und zweiten Typs, die zur Erfassung von akustischen Echos der übertragenen akustischen Wellen konfiguriert sind;
die erste Teilapertur empfängt ein erstes Signal mit einer ersten Phase und die zweite Teilapertur empfängt ein zweites Signal mit einer zweiten Phase; und,
eine Eingangsstufe mit einem Empfangs-Beamformer, die zur Erzeugung eines kombinierten Signals zur Bildgebung konfiguriert ist; das kombinierte Signal stammt von einer synthetisierten Apertur, die die erste und die zweite Teilapertur umfasst.

13. Vorrichtung nach Anspruch 12, wobei der Empfangs-Beamformer so konfiguriert ist, dass er das Bild durch Phasenanpassung des ersten Signals und des zweiten Signals erzeugt.

14. Vorrichtung nach Anspruch 13, wobei die Phasenanpassung des ersten Signals und des zweiten Signals das Anwenden einer ersten Verzögerung auf das erste Signal bzw. einer zweiten Verzögerung auf das zweite Signal umfasst, wobei die erste und die zweite Verzögerung zumindest teilweise auf der Grundlage einer Differenz zwischen einer ersten Laufzeit von einem oder mehreren Array-Elementen des ersten Typs (112) zu einem abzubildenden Medium und einer zweiten Laufzeit von einem oder mehreren Array-Elementen des zweiten Typs (114) zu dem Medium bestimmt werden.

15. Vorrichtung nach Anspruch 14, wobei die erste oder die zweite Verzögerung zumindest teilweise auf der Grundlage der Dicke und der Schallgeschwindigkeit einer akustischen Linse oder der Dicke und der Schallgeschwindigkeit einer akustischen Anpassungsschicht oder der Dicke und der Schallgeschwindigkeit sowohl einer akustischen Linse als auch einer akustischen Anpassungsschicht bestimmt wird.

16. Die Vorrichtung nach Anspruch 12, wobei der Empfangs-Beamformer so konfiguriert ist, dass er das Bild durch eines der folgenden Verfahren erzeugt:
a) Filtern des ersten Signals zur Rauschunterdrückung im ersten Signal und Filtern des zweiten Signals zur Rauschunterdrückung im zweiten Signal;
b) Verstärken des ersten Signals oder des zweiten Signals mit einer Verstärkung, um die Amplitude des ersten Signals und des zweiten Signals anzugleichen;
c) Anpassen der Frequenzen des ersten und des zweiten Signals; nach dem Anpassen der Frequenzen die Anpassung der Amplituden; und nach dem Anpassen der Frequenzen und Amplituden die Anpassung der Phasen.

17. Vorrichtung nach Anspruch 12, wobei das erste Signal eine Kombination von Signalen ist, die von einer Vielzahl von Array-Elementen des ersten Typs (112) stammen, oder das zweite Signal eine Kombination von Signalen ist, die von einer Vielzahl von Array-Elementen des zweiten Typs (114) stammen.

18. Vorrichtung nach Anspruch 12, wobei die Kombination des ersten Signals und des zweiten Signals eine kohärente Kombination ist.

19. Die Vorrichtung nach Anspruch 12, wobei die Eingangsstufe ferner so konfiguriert ist, dass das erste und das zweite Signal durch eine der folgenden Methoden kombiniert werden:
a) Auswählen der ersten Teilöffnung zur Übertragung akustischer Signale und Auswählen der ersten oder zweiten Teilöffnung zum Empfang akustischer Echos als Reaktion auf die akustischen Signale;
b) Auswählen eines Elements aus einem oder mehreren Array-Elementen des ersten Typs (112) zur Übertragung akustischer Signale und Auswählen der ersten Öffnung oder der zweiten Teilöffnung zum Empfang akustischer Echos als Reaktion auf die akustischen Signale.

## Revendications

1. Procédé d'imagerie acousto-optique comprenant :
la transmission d'ondes acoustiques ;
la réception d'un premier signal provenant d'une première sous-ouverture d'un réseau de capteurs ; la première sous-ouverture comprend un ou plusieurs éléments de réseau d'un premier type (112) ;
la réception d'un second signal provenant d'une seconde sous-ouverture du réseau de capteurs ; la seconde sous-ouverture comprend un ou plusieurs éléments de réseau d'un second type (114) différent du premier type ; le second type est un transducteur optoacoustique ; les éléments de réseau des premier et second types sont configurés pour détecter les échos acoustiques correspondant aux ondes acoustiques transmises ; et,
la combinaison, à l'aide d'un formateur de faisceaux de réception, du premier signal et du second signal pour obtenir un signal combiné permettant de former une image ; le signal combiné provient d'une ouverture synthétisée comprenant la première sous-ouverture et la seconde sous-ouverture du réseau de capteurs.

2. Procédé selon la revendication 1, comprenant en outre : l'alignement de phase du premier signal et du second signal.

3. Procédé selon la revendication 2, dans lequel l'alignement de phase du premier signal et du second signal comprend l'application d'un premier retard au premier signal ou d'un second retard au second signal, le premier retard et le second retard étant déterminés sur la base au moins en partie d'une différence entre un premier temps de propagation depuis le ou les éléments de réseau du premier type (112) vers un milieu étant imagé et un second temps de propagation depuis le ou les éléments de réseau du second type (114) vers le milieu.

4. Procédé selon la revendication 3, dans lequel le premier retard ou le second retard est déterminé sur la base au moins en partie d'une épaisseur et d'une vitesse acoustique d'une lentille acoustique, ou d'une épaisseur et d'une vitesse acoustique d'une couche d'alignement acoustique, ou d'une épaisseur et d'une vitesse acoustique de chacune parmi une lentille acoustique et une couche d'alignement acoustique.

5. Procédé selon la revendication 1, comprenant en outre l'une quelconque des étapes suivantes :
a) le filtrage du premier signal pour réduire le bruit dans le premier signal et le filtrage du second signal pour réduire le bruit dans le second signal ;
b) l'amplification du premier signal ou du second signal par un gain d'amplification pour aligner l'amplitude du premier signal et du second signal.

6. Procédé selon la revendication 2, dans lequel le premier signal est une combinaison de signaux provenant d'une pluralité d'éléments de réseau du premier type ou le second signal est une combinaison de signaux provenant d'une pluralité d'éléments de réseau du second type, ou les deux.

7. Procédé selon la revendication 6, comprenant en outre une ou plusieurs des étapes suivantes, avant l'alignement de phase du premier signal et du second signal : la génération du premier signal par combinaison de signaux provenant d'une pluralité d'éléments de réseau du premier type (112) ou d'une pluralité d'éléments de réseau du premier type et du second type (114) ; et la génération du second signal par combinaison de signaux provenant d'une pluralité d'éléments de réseau du second type, ou d'une pluralité d'éléments de réseau du premier type et du second type.

8. Procédé selon la revendication 7, comprenant en outre l'une quelconque des étapes suivantes :
a) la formation d'un élément de réseau effectif plus grand à partir d'une pluralité d'éléments de réseau du premier type (112), du second type (114) ou à la fois du premier et du second type ;
b) la réduction d'une dimensionnalité de l'ouverture synthétisée.

9. Procédé selon la revendication 1, comprenant en outre : l'alignement de fréquence du premier signal et du second signal ; après l'alignement de fréquence du premier signal et du second signal, l'alignement d'amplitude du premier signal et du second signal ; et après l'alignement de fréquence et l'alignement d'amplitude du premier signal et du second signal, l'alignement de phase du premier signal et du second signal.

10. Procédé selon la revendication 1, dans lequel la combinaison du premier signal et du second signal est une combinaison cohérente.

11. Procédé selon la revendication 1, comprenant en outre : la sélection de la première sous-ouverture pour la transmission de signaux acoustiques ; et la sélection de la première sous-ouverture ou de la seconde sous-ouverture pour la réception d'échos acoustiques en réponse aux signaux acoustiques.

12. Appareil permettant d'imager une cible, comprenant :
un transmetteur permettant de transmettre des ondes acoustiques ;
un ou plusieurs éléments de réseau d'un premier type (112) formant une première sous-ouverture ;
un ou plusieurs éléments de réseau d'un second type (114) différent du premier type et formant une seconde sous-ouverture, le second type étant un transducteur optoacoustique ;
les éléments de réseau des premier et second types configurés pour détecter les échos acoustiques correspondant aux ondes acoustiques transmises ;
la première sous-ouverture reçoit un premier signal ayant une première phase et la seconde sous-ouverture reçoit un second signal ayant une seconde phase ; et,
une partie frontale comprenant un formateur de faisceaux de réception et configurée pour obtenir un signal combiné permettant de former une image ; le signal combiné provient d'une ouverture synthétisée comprenant la première sous-ouverture et la seconde sous-ouverture.

13. Appareil selon la revendication 12, dans lequel le formateur de faisceaux de réception est configuré pour former l'image par alignement de phase du premier signal et du second signal.

14. Appareil selon la revendication 13, dans lequel l'alignement de phase du premier signal et du second signal comprend l'application d'un premier retard au premier signal ou d'un second retard au second signal, le premier retard et le second retard étant déterminés sur la base au moins en partie d'une différence entre un premier temps de propagation depuis le ou les éléments de réseau du premier type (112) vers un milieu étant imagé et un second temps de propagation depuis le ou les éléments de réseau du second type (114) vers ledit milieu.

15. Appareil selon la revendication 14, dans lequel le premier retard ou le second retard est déterminé sur la base au moins en partie d'une épaisseur et d'une vitesse acoustique d'une lentille acoustique, ou d'une épaisseur et d'une vitesse acoustique d'une couche d'alignement acoustique, ou d'une épaisseur et d'une vitesse acoustique de chacune parmi une lentille acoustique et une couche d'alignement acoustique.

16. Appareil selon la revendication 12, dans lequel le formateur de faisceaux de réception est configuré pour former l'image par l'une quelconque des étapes suivantes :
a) le filtrage du premier signal pour réduire le bruit dans le premier signal et le filtrage du second signal pour réduire le bruit dans le second signal ;
b) l'amplification du premier signal ou du second signal par un gain d'amplification pour aligner l'amplitude du premier signal et du second signal ;
c) l'alignement de fréquence du premier signal et du second signal ; après l'alignement de fréquence du premier signal et du second signal, l'alignement d'amplitude du premier signal et du second signal ; et après l'alignement de fréquence et l'alignement d'amplitude du premier signal et du second signal, l'alignement de phase du premier signal et du second signal.

17. Appareil selon la revendication 12, dans lequel le premier signal est une combinaison de signaux provenant d'une pluralité d'éléments de réseau du premier type (112) ou le second signal est une combinaison de signaux provenant d'une pluralité d'éléments de réseau du second type (114).

18. Appareil selon la revendication 12, dans lequel la combinaison du premier signal et du second signal est une combinaison cohérente.

19. Appareil selon la revendication 12, dans lequel la partie frontale est en outre configurée pour combiner le premier signal et le second signal par l'une quelconque des étapes suivantes :
a) la sélection de la première sous-ouverture pour la transmission de signaux acoustiques ; et la sélection de la première sous-ouverture ou de la seconde sous-ouverture pour la réception d'échos acoustiques en réponse aux signaux acoustiques ;
b) la sélection d'un élément parmi le ou les éléments de réseau du premier type (112) pour la transmission de signaux acoustiques ; et la sélection de la première ouverture ou de la seconde sous-ouverture pour la réception d'échos acoustiques en réponse aux signaux acoustiques.
